## Europäisches Patentamt

## European Patent Office

## Office européen des brevets

(11) Veröffentlichungsnummer: **0 219 728**
**B1**

(12) **EUROPÄISCHE PATENTSCHRIFT**

(45) Veröffentlichungstag der Patentschrift:
**23.08.89**

(51) Int. Cl.⁴: **A61K 31/615**

(21) Anmeldenummer: **86113453.4**

(22) Anmeldetag: **01.10.86**

(54) Kombinationspräparat.

(30) Priorität: **09.10.85 DE 3535950**

(43) Veröffentlichungstag der Anmeldung:
**29.04.87 Patentblatt 87/18**

(45) Bekanntmachung des Hinweises auf die Patenterteilung:
**23.08.89 Patentblatt 89/34**

(84) Benannte Vertragsstaaten:
**AT BE CH DE ES FR GB IT LI NL SE**

(73) Patentinhaber: **BASF Aktiengesellschaft,
Carl-Bosch-Strasse 38, D-6700 Ludwigshafen(DE)**

(72) Erfinder: **Lehmann, Hans Dieter, Prof. Dr., Im Hefen 15,
D-6945 Hirschberg/Leutershausen(DE)**
Erfinder: **Kretzschmar, Rolf, Prof. Dr., Oberer
Bergelweg 4, D-6718 Gruenstadt(DE)**
Erfinder: **Hahn, Klaus-Juergen, Prof. Dr.,
Mombertplatz 23, D-6900 Heidelberg(DE)**

(56) Entgegenhaltungen:
**ARZNEIMITTEL-FORSCHUNG, Band 23, Nr. 11,
November 1973, Seiten 1555-1560, Editio Cantor KG,
Aulendorf/Württ, DE; Horst KREISKOTT
"Tierexperimentelle Untersuchungen zur Hemmung der
Thrombozytenaggregation durch Verapamil in vitro und
in vivo"
THE LANCET, February 9, 1985, S. 351-352**

## Beschreibung

Die Erfindung betrifft ein neues Kombinationspräparat zur Behandlung von Krankheiten, die durch Thrombocytenaggregation ausgelöst werden.

Es ist bekannt, daß mit Verapamil, Gallopamil und Anipamil Herzkrankheiten, die durch eine mangelhafte Gewebsdurchblutung (Ischämie) verursacht sind, behandelt werden können (vgl. die Liste Pharamindex III/84, Isoptin® und Procorum® sowie EP-OS 64 158). Die Wirkung beruht sowohl auf einer gefäßdilatierenden als auch auf einer kardioprotektiven und einer thrombozytenaggregationshemmenden Wirkung. Es ist weiter bekannt, daß Acetylsalicylsäure zur Prophylaxe von thromboembolischen Erkrankungen aufgrund seiner thrombozytenaggregationshemmenden Wirkung verwendet wird (vgl. die Liste Pharmaindex III/84, Colfarit®).

Es wurde nun gefunden, daß sich die Wirkung von Verapamil, Gallopamil und Anipamil durch Acetylsalicylsäure stark erhöhen läßt.

Gegenstand der Erfindung ist ein Arzneimittel, dadurch gekennzeichnet, daß es einerseits Verapamil, Gallopamil oder Anipamil und andererseits Acetylsalicylsäure im Gewichtsverhältnis von 10 : 1 bis 1 : 20, vorzugsweise 7 : 1 bis 2 : 1, enthält.

Verapamil ist 1,7-Bis-(3,4-dimethoxyphenyl)-3-methylaza-7-cyan-8-methyl-nonan, Gallopamil ist 1-(3,4-Dimethoxyphenyl)-3-methylaza-7-cyan-7-(3,4,5--trimethoxyphenyl)-8-methyl-nonan und Anipamil ist 1,7-Bis-(3-methoxyphenyl)-3-methylaza-7-cyan-nonadecan.

In dem Arzneimittel können die Wirkstoffe in freier Form oder in Form ihrer physiologisch verträglichen Salze vorliegen. Zur Salzbildung mit den Verbindungen I eignen sich Salzsäure, Schwefelsäure, Phosphorsäure, Essigsäure, Malonsäure, Bernsteinsäure, Fumarsäure, Maleinsäure, Zitronensäure, Weinsäure, Milchsäure, Amidosulfonsäure und Oxalsäure. Als Salze der Acetylsalicylsäure kommen insbesondere die Alkali- und Erdalkalisalze in Betracht.

Wird die Mischung der Substanzen im Molverhältnis 1:1 eingesetzt, so können die Salze der Acetylsalicylsäure mit Verapamil, Gallopamil und Anipamil verwendet werden.

Bevorzugt werden für das neue Arzneimittel die Verbindungen I in Form ihrer Hydrochloride und die Acetylsalicylsäure in freier Form verwendet.

Das angegebene Mischungsverhältnis bezieht sich auf die freien Formen der Substanzen.

Die überlegene Wirkung der neuen Kombination läßt sich durch Bestimmung der Thrombozytenaggregation zeigen. Folgende Methodik wurde verwendet: Beagle-Hunde (12 bis 15 kg Körpergewicht) erhielten Verapamil und Acetylsalicylsäure einzeln und in Kombination auf oralem Wege. Vor sowie 2 und 4 h nach der Substanzgabe wurde venöses Blut entnommen, dieses durch Citrat-Zusatz ungerinnbar gemacht und daraus durch anschließende Zentrifugation (300 g, 10 min Dauer bei 4°C) thrombozytenreiches Plasma gewonnen. Zugabe von Adrenalin (Endkonzentration $5 \times 10^{-8}$ mol/l) und Kollagen (Endkonzentration $2 \times 10^{-3}$ g/l) zu thrombozytenreichem Plasma löst eine Aggregation aus, die als Extinktionsänderung im Born-Aggregometer MK 3 gemessen wurde. Als Aggregationsmaß fand die maximale Extinktionsänderung pro Sekunde Verwendung. Aus dem Vergleich der Werte vor und nach Gabe der Substanz wurde die prozentuale Hemmung der Aggregation ermittelt.

Unter diesen Bedingungen (siehe Tabelle) wird mit einer oralen Verapamil-Dosis von 0,1 mg/kg eine Aggregationshemmung um 27 % (2 h nach Substanzgabe) bzw. 17 % (4 h nach Substanzgabe) erzielt. Acetylsalicylsäure ist in einer Dosis von 1,0 mg/kg p.o. 2 und 4 h nach Gabe unwirksam (Hemmung 1,7 bzw. 2,8 %). Bei Kombination beider Substanzen in den vorgenannten Dosen tritt eine erhebliche Wirkungsverstärkung auf (44 bzw. 53 % Hemmung). Dieser Potenzierungseffekt ist überraschend und war nicht vorhersehbar. Die gefundene Wirkung bietet andererseits interessante Aspekte für die Therapie, da sie eine wertvolle Erweiterung der Verwendungsmöglichkeiten von Verapamil, Gallopamil und Anipamil sowie eine Dosisreduzierung bei der Anwendung von Acetylsalicylsäure möglich macht.

### Tabelle

Thrombozytenaggregationshemmende Wirkung von Verapamil, Acetylsalicylsäure und einer Kombination aus beiden nach oraler Zufuhr am Hund (N = 6)

| Zeitpunkt der Aggregationsmessung (h) | Aggregationshemmung (%) | | |
|---|---|---|---|
| | Verapamil (0,1 mg/kg p.o.) | Acetylsalicylsäure (1,0 mg/kg p.o.) | Verapamil (0,1 mg/kg p.o.) + Acetylsalicylsäure (1,0 mg/kg p.o.) |
| 2 | 27 | 1,7 | 44 |
| 4 | 17 | 2,8 | 53 |

Die neue Kombination eignet sich zur Behandlung von Prophylaxe von Erkrankungen, die durch Thrombocytenaggregation ausgelöst werden. Dazu gehören thromboembolische Erkrankungen im Bereich von Herz, Gehirn und peripherem, arteriellem Gefäßsystem. Solche Erkrankungen sind beispielsweise Herzinfarkt, Schlaganfall sowie obliterierende Arteriosklerose.

Die neue Kombination ist weiterhin geeignet zur Behandlung und Prophylaxe von Erkrankungen, die durch die Freisetzung vasoaktiver Stoffe aus aggregierenden Thrombocyten hervorgerufen werden, wie Migräne, vasospastische Angina und Morbus Raynaud.

Die erfindungsgemäße Kombination kann in üblicher Weise oral verabfolgt werden.

Die Dosierung hängt vom Alter, Zustand und Gewicht des Patienten sowie von der Applikationsart ab. In der Regel beträgt die tägliche Dosis zwischen etwa 20 und 500 mg, vorzugsweise zwischen 50 und 200 mg, Verapamil, Gallopamil bzw. Anipamil und zwischen etwa 10 und 300, vorzugsweise zwischen 50 und 200 mg, Acetylsalicylsäure pro Patient und Tag.

Die neue Kombination kann in den gebräuchlichen galenischen Applikationsformen fest oder flüssig angewendet werden, z.B. als Tabletten, Filmtabletten, Kapseln, Pulver, Granulate, Dragees, Pellets, Retardpellets oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 10 bis 99 Gewichtsprozent.

## Beispiel 1

Auf einer Tablettenpresse werden in üblicher Weise Tabletten folgender Zusammensetzung gepreßt:
240 mg Verapamilhydrochlorid
50 mg Acetylsalicylsäure
72 mg Maisstärke
13 mg Gelatine
35 mg Milchzucker
25 mg Carboxymethylcellulose
17,5 mg Talcum
3,5 mg Magnesiumstearat

## Beispiel 2

In üblicher Weise werden Drageekerne folgender Zusammensetzung hergestellt:
50 mg Gallopamil
50 mg Acetylsalicylsäure
100 mg Kernmasse
Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60 : 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk.

Die erhaltenen Kerne werden anschließend mit einer Verzuckerungsmasse folgender Zusammensetzung überzogen:
5 Teile Saccharose 2 Teile Maisstärke
2 Teile Calciumcarbonat
1 Teil Talk

## Beispiel 3

Zur Herstellung von Filmtabletten werden die gemäß Beispiel 2 hergestellten Drageekerne mit einem Filmüberzug folgender Zusammensetzung überzogen:

| Tylose | 0,7% | Maisstärke | 2,0% |
|---|---|---|---|
| ®Kollidon 25 (PVP) | 0,4% | Calciumcarbonat | 3,5% |
| Saccharose | 70,0% | Gummi arabicum | 2,5% |
| hochdisperse Kieselsäure | 1,4% | Titandioxid + Farbstoffe | 8,5% |
| Talcum | 11,0% | | |

Beispiel 4

Es werden in Steckkapseln abfüllbare Retardpellets hergestellt, wobei jede Komponente für sich pelletiert und die Retardpellets anschließend gemischt oder nacheinander in die Kapseln gefüllt werden.
Zusammensetzung der Pellets pro Dosis.

Retardpellets, Verbindung I: 5O mg Gallopamilhydrochlorid
6O mg Cellulosepulver
5 mg Maisstärke
1O mg Talkum
35 mg Ethylcellulose

Retardpellets, Verbindung II: 1OO mg Acetylsalicylsäure
2O mg Cellulosepulver
1O mg Maisstärke
1O mg Ethylcellulose

Beispiel 5 Es werden in Steckkapseln abfüllbare Pellets hergestellt mit folgender Zusammensetzung:
1OO mg Verapamil
1OO mg Acetylsalicylsäure
8O mg Cellulosepulver
3O mg Maisstärke
5 mg ®Kollidon 3O (PVP)
2O mg ®Eudragit S (Polymerisat aus Methylacrylsäure und Methacrylsäureester 15 mg Talkum

**Patentansprüche**

1. Arzneimittel, dadurch gekennzeichnet, daß es einerseits Verapamil, Gallopamil oder Anipamil und andererseits Acetylsalicylsäure im Gewichtsverhältnis von 1O : 1 bis 1 : 2O enthält.
2. Arzneimittel, gemäß Anspruch 1, dadurch gekennzeichnet, daß es Verapamil und Acetylsalicylsäure enthält.
3. Verfahren zur Herstellung eines Arzneimittels, dadurch gekennzeichnet, daß man eine Mischung aus einerseits Verapamil, Gallopamil oder Anipamil und andererseits Acetylsaliylsäure im Gewichtsverhältnis von 1O : 1 bis 1 : 2O in eine galenische Applikationsform einarbeitet.

**Claims**

1. A drug which contains, on the one hand, verapamil, gallopamil or anipamil and, on the other hand, acetylsalicylic acid in a weight ratio of from 10:1 to 1:20.
2. A drug as claimed in claim 1, which contains verapamil and acetylsalicylic acid.
3. A process for the preparation of a drug, which comprises incorporating a mixture of, on the one hand, verapamil, gallopamil or anipamil and, on the other hand, acetylsalicylic acid in a weight ratio of from 10:1 to 1:20 into a pharmaceutical administration form.

**Revendications**

1. Médicament caractérisé par le fait qu'il contient, d'une part, du vérapamile, du gallopamile et de l'anipamile et, d'autre part, de l'acide acétylsalicylique en proportion en poids de 10/1 à 1/20.
2. Médicament selon la revendication 1, caractérisé par le fait qu'il contient du vérapamile et de l'acide acétylsalicylique.
3. Procédé de préparation d'un médicament, caractérisé par le fait qu'on met sous forme d'application galvanique un mélange, d'une part, du vérapamile, du gallopamile et de l'anipamile et, d'autre part, de l'acide acétylsalicylique en proportion en poids de 10/1 à 1/20.